# EUROPEAN PATENT APPLICATION

(11) **EP 4 813 219 A1**
(43) Date of publication of application: **30.09.2026**
(21) Application number: 25878443.8
(22) Date of filing: 19.11.2025
(51) Int. Cl.: A23L 33/105, A61P 35/00

(54) **COMPOSITION FOR ALLEVIATING FATIGUE OR ENHANCING EXERCISE PERFORMANCE**

(30) Priority: 19.11.2024 KR 20240165331
(71) Applicant: Kolmar BNH Co., Ltd, Sejong 30003 (KR)
(72) Inventor: BAK, Su Bin, Seoul 06800 (KR); SHIN, Ji Eun, Seoul 06800 (KR); KIM, Ju Gyeong, Seoul 06800 (KR); KIM, Sang Back, Seoul 06800 (KR); KIM, Young Mu, Seoul 06800 (KR)
(74) Representative: Regimbeau
(86) International application number: PCT/KR2025/019195
(87) International publication number: WO 2026/111406

(57) **Abstract**

The present disclosure relates to a composition for alleviating fatigue or enhancing exercise performance, comprising, as an active ingredient, a mixture of a mixed extract of angelica, cnidium, and paeonia and a polysaccharide isolate thereof; or a fraction thereof.

## Description

### [Technical Field]

The present disclosure relates to a composition for alleviating fatigue or enhancing exercise performance, comprising, as an active ingredient, a mixture of a mixed extract of angelica, cnidium, and paeonia and a polysaccharide isolate thereof; or a fraction thereof.

### [Background Art]

Although the average lifespan of Koreans has increased, individuals are increasingly exposed to various adult diseases due to stress and fatigue, and many suffer from chronic fatigue caused by imbalances in biological rhythms resulting from mental overload. The dictionary definition of fatigue refers to lethargy or weariness resulting from physical or mental effort. Specifically, fatigue refers to an abnormal exhaustion after routine activities, a state in which a lack of energy prevents continuous effort or tasks requiring concentration, or a general lack of vitality severe enough to impede the performance of ordinary daily activities, due to a temporary decline in the strength or sensitivity of cells, muscles, or organs following sustained activity or stimulation.

Fatigue is distinguished from a disease (or disorder) in which an organism suffers from a state of abnormal physical or mental functionality. A more narrowly defined form of fatigue refers generally to physical fatigue leading to reduced work capacity, and is also distinguished from stress, in which mental fatigue causes disruption in homeostasis (see Ministry of Food and Drug Safety (MFDS), Establishment of Functionality Evaluation System for Fatigue Recovery of Health Functional Foods, Guideline for Functionality Evaluation of Health Functional Foods (in relation to Fatigue Alleviation)).

If fatigue persists, it may progress to chronic fatigue syndrome, which, unlike transient fatigue that is relieved by brief rest, is characterized by persistent fatigue that does not improve with rest and causes significant debilitation in the patient. However, the underlying cause of this condition has not yet been clearly identified.

Recovery from physical fatigue requires an adequate supply of energy and rest, as well as the suppression and elimination of fatigue-inducing substances from the body. However, in today's fast-paced society, sufficient nutritional intake and proper rest are often not achieved. Consequently, extensive research utilizing natural products for fatigue recovery, enhancement of endurance, and improvement of immune function has recently been actively pursued in both academia and industry.

Meanwhile, muscle strength refers to the maximum force that muscles or muscular tissues can exert, and exercise performance refers to the ability to use such muscle strength to perform physical movements in daily life or sports quickly, powerfully, for an extended duration, and with proficiency. When muscles are lost due to congenital physical disabilities or acquired reasons such as prolonged hospitalization, muscle strength decreases and exercise performance is reduced, and accordingly, enhancement of exercise performance becomes necessary. For athletes, enhancing exercise performance is required to improve competitive performance.

Methods used for enhancing exercise performance include scientific training tailored to the type of sport, dietary regimens, improvements in technical equipment, ergogenic aids, *etc*. Among these methods, research related to functional supplements for improving exercise performance has been actively conducted worldwide. However, many ergogenic aids used in Western countries, such as caffeine and anabolic steroids, are associated with adverse side effects and pose risks of doping violations. Although recent research has actively pursued the development of functional supplements using natural products with assured safety, such research remains insufficient. Therefore, there is a need for research on supplements that can effectively enhance exercise performance without causing adverse side effects.

### [Disclosure]

### [Technical Problem]

An object of the present disclosure is to provide a composition for alleviating fatigue or enhancing exercise performance, comprising, as an active ingredient, a mixture of a mixed extract of angelica, cnidium, and paeonia; and a polysaccharide isolate thereof.

Another object of the present disclosure is to provide a method for alleviating fatigue or enhancing exercise performance, comprising administering to a subject a composition comprising, as an active ingredient, a mixture of a mixed extract of angelica, cnidium, and paeonia; and a polysaccharide isolate thereof.

Still another object of the present disclosure is to provide a use of a composition comprising, as an active ingredient, a mixture of a mixed extract of angelica, cnidium, and paeonia; and a polysaccharide isolate thereof for alleviating fatigue or enhancing exercise performance.

### [Technical Solution]

An aspect of the present disclosure is a composition for alleviating fatigue or enhancing exercise performance, comprising, as an active ingredient, a mixture of a mixed extract of angelica, cnidium, and paeonia; and a polysaccharide isolate thereof.

In one specific embodiment, the fatigue is characterized by comprising physical fatigue, muscle fatigue, or a combination thereof.

In another specific embodiment, the mixed extract is characterized by being extracted with a solvent selected from the group consisting of water, an organic solvent, and a mixed solvent thereof.

In a specific embodiment according to any one of the preceding specific embodiments, the mixed extract is characterized by comprising angelica, cnidium, and paeonia at a weight ratio of 1:1.4 to 3.5:1.2 to 3.

In a specific embodiment according to any one of the preceding specific embodiments, the mixed extract is characterized by being a mixed extract of *Angelica sinensis* (Oliv.) Diels, *Ligusticum chuanxiong* Hort. or *Cnidium officinale*, and *Paeonia lactiflora* Pallas.

In a specific embodiment according to any one of the preceding specific embodiments, the polysaccharide isolate is characterized by being an ethanol polysaccharide isolate.

In a specific embodiment according to any one of the preceding specific embodiments, the ethanol is characterized by being 60% to 100% ethanol.

In a specific embodiment according to any one of the preceding specific embodiments, the mixture is characterized by comprising the mixed extract of angelica, cnidium, and paeonia and the polysaccharide isolate thereof at a ratio of 60 to 70:30 to 40.

In a specific embodiment according to any one of the preceding specific embodiments, the mixture is characterized by being comprised in an amount of 0.001% to 90% by weight based on the total weight of the composition.

In a specific embodiment according to any one of the preceding specific embodiments, the composition can increase expression of heme oxygenase-1 (HO-1).

In a specific embodiment according to any one of the preceding specific embodiments, the composition can activate a nuclear factor erythroid 2-related factor-2 (Nrf-2) pathway.

In a specific embodiment according to any one of the preceding specific embodiments, the composition can increase expression of one or more selected from the group consisting of SOD, CAT, Txn, Keap1, NQO1, GPx, Gclc, and GSr.

In a specific embodiment according to any one of the preceding specific embodiments, the composition is characterized by being a food composition.

In a specific embodiment according to any one of the preceding specific embodiments, the food composition is characterized by being prepared as a powder, granule, tablet, capsule, syrup, or beverage.

In a specific embodiment according to any one of the preceding specific embodiments, the food can be a health functional food.

Another aspect of the present disclosure is a method for alleviating fatigue or enhancing exercise performance, comprising administering to a subject a composition comprising, as an active ingredient, a mixture of a mixed extract of angelica, cnidium, and paeonia and a polysaccharide isolate thereof.

Still another aspect of the present disclosure is a use of a composition comprising, as an active ingredient, a mixture of a mixed extract of angelica, cnidium, and paeonia; and a polysaccharide isolate thereof for alleviating fatigue or enhancing exercise performance.

### [Advantageous Effects]

The composition comprising, as an active ingredient, a mixture of a mixed extract of angelica, cnidium, and paeonia and a polysaccharide isolate thereof; or a fraction thereof according to the present disclosure can effectively alleviate accumulated physical fatigue and enhance exercise performance by modulating various factors involved in physical fatigue and/or enhancement of exercise performance in various ways, and can be thus applied as a food composition for alleviating fatigue or enhancing exercise performance.

### [Brief Description of Drawings]

FIG. 1 illustrates the antioxidant activity evaluated by treating L6 skeletal muscle cells with a composition according to an example of the present disclosure.
FIG. 2 illustrates the fatigue-enhancing effect evaluated by measuring ATP and glycogen contents in C2C12 myotube cells treated with a composition according to an example of the present disclosure at varying concentrations.
FIG. 3 illustrates a comparison of ATP contents in C2C12 myotube cells treated with a composition according to an example of the present disclosure and compositions of Comparative Examples 1 to 6.
FIG. 4 illustrates a comparison of glycogen contents in C2C12 myotube cells treated with a composition according to an example of the present disclosure and compositions of Comparative Examples 1 to 6.
FIG. 5 illustrates expression levels of glycogen synthase (GS) and glycogen phosphorylase (GP) in C2C12 myotube cells treated with a composition according to an example of the present disclosure at varying concentrations.
FIG. 6 illustrates rotarod test results in mouse models administered with a composition according to an example of the present disclosure.
FIG. 7 illustrates forced swimming test results in mouse models administered with a composition according to an example of the present disclosure.
FIG. 8 illustrates glucose contents in blood of mouse models administered with a composition according to an example of the present disclosure.
FIG. 9 illustrates creatine kinase (CK) contents in blood of mouse models administered with a composition according to an example of the present disclosure.
FIG. 10 illustrates glycogen contents in muscular tissue of mouse models administered with a composition according to an example of the present disclosure.
FIG. 11 illustrates antioxidant factors (NRf-2 and HO-1) analyzed by RT-qPCR in tissue of mouse models administered with a composition according to an example of the present disclosure.
FIG. 12 illustrates antioxidant factors (NRf-2 and HO-1) analyzed by western blot in tissue of mouse models administered with a composition according to an example of the present disclosure.
FIG. 13 illustrates immunohistochemical staining of calf muscle tissues of mouse models administered with a composition according to an example of the present disclosure.

### [Modes for Carrying Out the Invention]

Each description and embodiment disclosed in the present disclosure may also be applied to other descriptions and embodiments. That is, all combinations of various elements disclosed in the present disclosure fall within the scope of the present disclosure. Further, the scope of the present disclosure is not limited by the specific description described below.

Furthermore, those skilled in the art will recognize or be able to ascertain, using no more than routine experimentation, many equivalents to the specific aspects of the present disclosure described herein. Additionally, such equivalents are intended to be included in the present disclosure.

Furthermore, a number of papers and patent documents are referenced and cited throughout the present disclosure. The disclosures of the cited papers and patent documents are incorporated herein by reference in their entirety to further clarify the level of the art and the description of the present disclosure.

In addition, throughout the specification of the present disclosure, when a part is said to "comprise" a certain component, this does not mean that other components are excluded unless otherwise specifically stated, but rather that other components may be further included.

As used herein, the term "about" may be presented before a specific numerical value and encompasses not only the exact numerical value that follows the term, but also values that are approximately that number or fall within a range close to it. Considering the context in which the number is presented, it can be determined whether a value is close to, or approximately equal to, the specific numerical value. In one example, the term "about" may refer to a range of -10% to +10% of a numerical value. In another example, the term "about" may refer to a range of -5% to +5% of a given numerical value. However, the term is not limited thereto.

Hereinafter, the present disclosure is described in more detail.

The present disclosure is based on the discovery that a mixture of a mixed extract of angelica, cnidium, and paeonia; and a polysaccharide isolate thereof activates an Nrf-2 pathway and increases HO-1 expression, thereby modulating various factors involved in physical fatigue and/or enhancement of exercise performance in various ways to effectively alleviate accumulated physical fatigue and enhance exercise performance.

An aspect of the present disclosure to achieve the above object provides a composition for alleviating fatigue or enhancing exercise performance, comprising, as an active ingredient, a mixture of a mixed extract of angelica, cnidium, and paeonia and a polysaccharide isolate thereof; or a fraction thereof.

As used herein, the term "angelica" refers to an angiosperm belonging to the *Apiaceae* family, which has long been used medicinally in East Asia, with its root portion most commonly utilized. Accordingly, the dried root of angelica is also interchangeably referred to as "angelica".

Depending on the origin, *Angelica sinensis* (Oliv.) Diels derived from China is distinguished from *Angelica gigas* Nakai in Korea and *Angelica acutiloba* (Siebold. & Zucc.) Kitag. or *Angelica acutiloba* (Siebold. & Zucc.) Kitag. var. sugiyamae Hikino in Japan.

*Angelica sinensis* is warm in energy and has a sweet and spicy taste. In general, *Angelica sinensis* is sweeter and less spicy than *Angelica gigas*. The efficacy of *Angelica sinensis* is to replenish blood when there is a lack of blood, and angelica made of the root of *Angelica sinensis* has an excellent blood-replenishing effect. However, angelica made of the root of *Angelica gigas* is more effective in promoting blood circulation than in replenishing blood, and has strong anticancer and blood pressure-lowering effects. Pharmacologically, it is known that angelica promotes blood flow in coronary arteries and stimulates red blood cell production.

As used herein, the term "cnidium" include*s Ligusticum chuanxiong* Hort. or *Cnidium officinale* depending on the origin, and refers to a perennial herb of the *Apiaceae* family, *Apiaceae* order, and *Dicotyledonous* group of dicotyledons commonly cultivated as a medicinal plant. Cnidium is effective for sedation, pain relief, and strengthening, and is used to treat headaches, anemia, and gynecological diseases. The rhizome of cnidium is dug up in September to November, dried in the sun with the leaves and stems removed, and then boiled and taken or made into pills or powders for use.

As used herein, the term "paeonia (*Paeonia lactiflora* Pallas)" refers to a perennial herb of the *Paeonia* genus in the *Paeoniaceae* family, a dicotyledonous plant growing in mountainous areas, and is used for horticultural purposes because of its beautiful flowers. Additionally, the root is used as a medicinal herb for pain, abdominal pain, menstrual cramps, amenorrhea, vomiting blood, anemia, and bruises. Paeonia has been cultivated as an ornamental plant in China since the Qin and Ming Dynasties, and its cultivation history is longer than that of *Paeonia suffruticosa* Andr. Dozens of varieties of Paeonia were recorded during the Song and Qing Dynasties, and are distributed in Korea, Mongolia, and East Siberia.

In the present disclosure, paeonia may include one or more selected from the group consisting of *Paeonia lactiflora* Pallas, *Paeonia obovata var. japonica*, *Paeonia* sect. *Paeonia*, *Paeonia lactiflora for. pilosella Nakai*, and *Paeonia lactiflora var. trichocarpa (Bunge) Stern*, and may be used without limitation regardless of the processing method.

The angelica, cnidium, and paeonia may be purchased commercially, collected from nature, or cultivated and used without limitation.

As used herein, the term "extract" refers to a mixed extract containing angelica, cnidium, and paeonia, and the mixed extract of angelica, cnidium, and paeonia may be obtained from various organs of natural, hybrid, and mutant plants. Specifically, the extract may be extracted from roots, aerial parts, stems, leaves, flowers, fruit bodies, and fruit peels as well as plant tissue cultures.

The "mixed extract" is a mixed extract comprising all of angelica, cnidium, and paeonia, and may be an extract obtained by first mixing the three substances and then subjecting the mixture to extraction, or may be an extract obtained by subjecting each substance to extraction individually and then mixing the extracts thus prepared. The term also includes, without limitation, any mixed extract prepared by any other known method capable of preparing a mixed extract.

In one embodiment of the present disclosure, the extract includes extracts in all formulations that can be formed from the extract itself or using the extract, such as an extract obtainable by extracting angelica, cnidium, and paeonia; a diluted or concentrated extract; a dried product obtained by drying an extract; modified or purified products thereof; or mixtures thereof.

The extract may be extracted with a solvent selected from the group consisting of water, an organic solvent, and a mixed solvent thereof, but is not limited thereto.

In a case where the solvent of the extract is water, the extract may include a cold water extract or a hot water extract.

In one specific embodiment, the organic solvent may be a linear or branched alcohol having 1 to 6 carbon atoms; specifically one or more selected from the group consisting of methanol, ethanol, propanol, butanol, pentanol, and hexanol; and more specifically ethanol, but is not limited thereto.

In one specific embodiment, the organic solvent may be glycerol, ethylene glycol, propylene glycol, hydrocarbon-based solvents such as methyl acetate, ethyl acetate, benzene, n-hexane, diethyl ether, dichloromethane, and chloroform, or nonpolar organic solvents such as petroleum ether, methyl acetate, benzene, hexane, chloroform, methylene chloride, dimethyl ether, and ethyl acetate, but is not limited thereto.

The solvent may also include an aqueous solution of an organic solvent. The concentration thereof is not particularly limited, but may be 1% to 99% (v/v), specifically 60% to 98% (v/v), more specifically 80% to 95% (v/v), and still more specifically 95% (v/v).

In one embodiment of the present disclosure, the extract may be an extract obtained by performing extraction in an extraction temperature range of 20°C to 100°C for an extraction period of about 1 hour to 10 days by an extraction method such as hot water extraction, cold extraction, reflux cooling extraction, or ultrasonic extraction. However, the extraction is not limited to the extraction temperature, extraction period, or extraction method above.

The extract may contain angelica, cnidium, and paeonia at a weight ratio of 1 to 10:1 to 10:1 to 10, for example, a weight ratio of 1 to 5:1 to 5:1 to 5, a weight ratio of 1:1.4 to 3.5:1.2 to 3, 1:1.5 to 3:1.3 to 2.5, 1:1.5 to 2:1.3 to 1.6, 1:1.5 to 1.8:1.3 to 1.5, or a weight ratio of 1:1.6:1.3, but the weight ratio is not limited thereto.

In one specific embodiment, the extract may be a mixed extract comprising *Angelica sinensis* (Oliv.) Diels, *Ligusticum chuanxiong* Hort. or *Cnidium officinale*, and *Paeonia lactiflora* Pallas at a weight ratio of 1:1.6:1.3.

In one specific embodiment, the extract may be a mixed extract comprising *Angelica sinensis* (Oliv.) Diels, *Ligusticum chuanxiong* Hort., and *Paeonia lactiflora* Pallas at a weight ratio of 1:1.6:1.3.

To the extract of the present disclosure, an extract of a known natural product or a known component that alleviates fatigue or enhances exercise performance may optionally be added.

As used herein, the term "polysaccharide isolate" includes a polysaccharide precipitated by adding a solvent to the mixed extract of angelica, cnidium, and paeonia. Here, the polysaccharide is also called a polysaccharide and may comprise one or more polysaccharides.

In the present disclosure, the method for obtaining the polysaccharide isolate is not particularly limited, and may be performed according to a method commonly used in the art. As a non-limiting example of the method, the polysaccharide isolate may be obtained from the extract of angelica, cnidium, and paeonia of the present disclosure by treating the extract with a predetermined solvent. In one example, the polysaccharide isolate may be produced by adding ethanol to the extract of angelica, cnidium, and paeonia of the present disclosure, but the method is not limited thereto.

In the present disclosure, the type of solvent used to obtain the polysaccharide isolate is not particularly limited, and any solvent known in the art may be used. Non-limiting examples of the solvent include water, an organic solvent, or a mixed solvent thereof. These may be used alone or in a mixture of two or more, but the solvent is not limited thereto.

In one specific embodiment, the organic solvent may be a linear or branched alcohol having 1 to 6 carbon atoms, specifically one or more selected from the group consisting of methanol, ethanol, propanol, butanol, pentanol, and hexanol, and more specifically ethanol, but is not limited thereto.

Further, the organic solvent may be one or more selected from the group consisting of dichloromethane, diethyl ether, chloroform, and ethyl acetate, but is not limited thereto.

In one embodiment of the present disclosure, the polysaccharide isolate may be an ethanol polysaccharide isolate, but is not limited thereto.

In one specific embodiment, the ethanol polysaccharide isolate may be a 60% to 100% ethanol polysaccharide isolate, 70% to 100% ethanol polysaccharide isolate, 80% to 100% ethanol polysaccharide isolate, 85% to 100% ethanol polysaccharide isolate, 90% to 100% ethanol polysaccharide isolate, 95% to 100% ethanol polysaccharide isolate, 80% to 95% ethanol polysaccharide isolate, or 85% to 95% ethanol polysaccharide isolate, and specifically a 60%, 70%, 80%, 85%, 90%, 95%, or 100% ethanol polysaccharide isolate, but is not limited thereto.

As used herein, the term "mixture" refers to a mixture in which the extracts of angelica, cnidium, and paeonia prepared in the present disclosure are mixed with polysaccharide isolates obtained by treating each of the extracts with a predetermined solvent, and the mixing ratio thereof is not particularly limited.

The composition uses extracts of each plant and polysaccharide isolates thereof in combination, and can thereby achieve remarkably superior effects compared to cases comprising only an extract of one plant and a polysaccharide isolate thereof or a polysaccharide thereof. In addition, the composition can exhibit a remarkably superior immunity enhancing or strengthening effect compared to cases comprising extracts of plants other than angelica, cnidium, and paeonia, polysaccharide isolates thereof, or polysaccharides thereof.

When the mixture is a mixture of an extract and a polysaccharide isolate, the extract and the polysaccharide isolate may be comprised at a weight ratio of about 40 to 80:20 to 60, for example, a weight ratio of 45 to 75:25 to 55, 50 to 70:30 to 50, or 60 to 70:30 to 40, and specifically a weight ratio of 60:40, but the weight ratio is not limited thereto.

As used herein, the term "fraction thereof" refers to a product obtained by performing fractionation to isolate a specific component or a specific component group from a mixture comprising various components.

In the present disclosure, the fractionation method to obtain the fraction is not particularly limited, and may be performed according to a method commonly used in the art. As a non-limiting example of the fractionation method, the fraction may be a fraction obtained by treating a mixture in which the extract of angelica, cnidium, and paeonia of the present disclosure and a polysaccharide thereof are mixed with a predetermined solvent.

In the present disclosure, the type of fractionation solvent used to obtain the fraction is not particularly limited, and any solvent known in the art may be used. Non-limiting examples of the fractionation solvent include water, an organic solvent, or a mixed solvent thereof. These may be used alone or in a mixture of one or more, but the solvent is not limited thereto. In addition, the organic solvent may be one or more selected from the group consisting of a linear or branched alcohol having 1 to 6 carbon atoms, dichloromethane, diethyl ether, chloroform, and ethyl acetate, but is not limited thereto.

Specifically, the fraction may be a fraction produced by adding water or ethanol to a mixture in which the extract of angelica, cnidium, and paeonia of the present disclosure and a polysaccharide isolate thereof are mixed, and more specifically, a water fraction or ethanol fraction of a mixture in which the extract of angelica, cnidium, and paeonia and a polysaccharide isolate thereof are mixed.

More specifically, the fraction may be a water fraction of a mixture in which the extract of angelica, cnidium, and paeonia and a polysaccharide isolate thereof are mixed.

The mixture of the extract of angelica, cnidium, and paeonia and a polysaccharide isolate thereof, or a fraction thereof, may be comprised in an amount of 0.0001% to 90% by weight based on the total weight of the composition. Specifically, the mixture or a fraction thereof may be comprised in an amount of 30% to 80% by weight, more specifically 35% to 70% by weight, and still more specifically 40% to 65% by weight, based on the total weight of the composition, but is not limited thereto.

The mixture of the extract of angelica, cnidium, and paeonia and a polysaccharide isolate thereof, or a fraction thereof, can activate a nuclear factor erythroid 2-related factor-2 (Nrf-2) pathway, or increase expression of heme oxygenase-1 (HO-1).

The term "HO-1" is known as a representative cellular defensive phase 2 detoxifying antioxidant enzyme, and the induction of HO-1 functions as an important mechanism against various diseases or conditions associated with oxidative stress or inflammatory tissue damage.

In addition, "Nrf-2" is a critical transcription factor that protects cells against oxidative stress, carcinogenic processes, *etc*., and activates the transcription of antioxidant enzymes and phase 2 detoxifying enzymes (Nutrients 2018, 10, 858).

Accordingly, the mixture of the extract and polysaccharide isolate, or a fraction thereof, activates a nuclear factor erythroid 2-related factor-2 (Nrf-2) pathway or increases heme oxygenase-1 (HO-1) expression, thereby modulating various factors involved in physical fatigue and/or enhancement of exercise performance to effectively achieve effects of alleviating physical fatigue and/or enhancing exercise performance.

Furthermore, the composition may increase expression of one or more antifatigue indicators selected from the group consisting of superoxide dismutase (SOD), catalase (CAT), thioredoxin (Txn), Kelch-like ECH-associated protein 1 (Keap1), NAD(P)H quinone dehydrogenase 1 (NQO1), glutathione peroxidase (GPx), glutamate-cysteine ligase catalytic subunit (Gclc), and glutathione reductase (GSr).

For example, the composition may increase movement distance and exercise time in behavioral tests, thereby demonstrating enhanced exercise performance. In addition, the composition may increase ATP and glycogen concentrations in muscle. Furthermore, the composition may increase the concentrations or expression levels of glycogen synthase (GS) and glycogen phosphorylase (GP).

As used herein, the term "improvement" means alleviating fatigue or enhancing, augmenting, or strengthening exercise performance by applying the mixture of the extract of angelica, cnidium, and paeonia and a polysaccharide isolate thereof, or a fraction thereof, to a composition.

The composition comprising the mixture of the extract of angelica, cnidium, and paeonia and a polysaccharide isolate thereof, or a fraction thereof, may be added to a food composition for the purpose of alleviating fatigue or improving, augmenting, or strengthening exercise performance, and the food composition may be a health functional food composition.

The food composition may contain a sitologically acceptable carrier.

The food composition of the present disclosure includes all forms such as functional food, nutritional supplement, health food, and food additives, and the above types of food composition may be prepared in various forms according to conventional methods known in the art.

In a case where the mixture of the extract of angelica, cnidium, and paeonia and a polysaccharide isolate thereof, or a fraction thereof, is used as a food additive, the mixture of the extract of angelica, cnidium, and paeonia and a polysaccharide isolate thereof, or a fraction thereof may be added as is or may be used together with other foods or food ingredients, and may be used appropriately according to conventional methods. The amount of active ingredients mixed may be suitably determined depending on the purpose of use (prevention, health, or therapeutic treatment). Generally, when a food or beverage is prepared, the mixture of the extract of angelica, cnidium, and paeonia and a polysaccharide isolate thereof, or a fraction thereof is added to the raw material composition in an amount of 0.0001% to 90% by weight, preferably 0.001% to 50% by weight. However, in the case of long-term intake for health and hygiene purposes or health control purposes, the amount may be below the above range.

The type of food is not particularly limited. Examples of the food to which the substances can be added include meat, sausage, bread, chocolate, candy, snacks, confectionery, pizza, ramen, other noodles, gum, dairy products including ice cream, various soups, beverages, tea, drinks, alcoholic beverages, and vitamin complexes, and may include all health foods in the conventional sense.

The health beverage composition of the present disclosure may contain various flavoring agents, natural carbohydrates, *etc*. as additional ingredients, like conventional beverages. The natural carbohydrates described above are monosaccharides such as glucose and fructose, disaccharides such as maltose and sucrose, polysaccharides such as dextrin and cyclodextrin, and sugar alcohols such as xylitol, sorbitol, and erythritol. As sweeteners, natural sweeteners such as thaumatin and stevia extract or synthetic sweeteners such as saccharin and aspartame may be used. The proportion of the natural carbohydrates may be generally about 0.001 to 50 parts by weight, specifically about 0.01 to 30 parts by weight, per 100 parts by weight of the composition of the present disclosure.

In addition to the above, the composition of the present disclosure may contain various nutrients, vitamins, electrolytes, flavoring agents, coloring agents, pectic acid and its salts, alginic acid and its salts, organic acids, protective colloid thickeners, pH adjusters, stabilizers, preservatives, glycerin, alcohols, carbonating agents used in carbonated beverages, *etc*. The proportion of these additives is not greatly important, but is generally selected in the range of 0.01 to 30 parts by weight per 100 parts by weight of the composition of the present disclosure. In addition, the composition of the present disclosure may contain fruit pulp for the preparation of natural fruit juice, fruit juice beverages, and vegetable beverages. The proportion of the pulp is not greatly important, but is generally selected in the range of 0.01 to 30 parts by weight per 100 parts by weight of the composition of the present disclosure, and these ingredients may be used independently or in combination.

In one embodiment, the food may be a health functional food.

As used herein, the term "health functional food" refers to a food prepared and processed using raw materials or ingredients having functionality beneficial to the human body, and "functionality" means achieving effects useful for health-related use, such as regulating nutrients or exerting physiological actions with regard to the structure and functions of the human body. Meanwhile, health functional food refers to a food having active effects for maintaining or promoting health compared to general foods, and health supplementary food refers to a food for health supplementation. In some cases, the terms "health functional food," "health food," and "health supplementary food" may be used interchangeably.

In one embodiment of the present disclosure, the composition may be a quasi-drug composition comprising a mixture of an extract of angelica, cnidium, and paeonia and a polysaccharide isolate thereof, or a fraction thereof, for alleviating fatigue or improving, augmenting, or strengthening exercise performance.

The mixed extract, polysaccharide isolate, mixture, and fraction are as described above.

The quasi-drug of the present disclosure refers to articles, among those used for the purpose of diagnosing, curing, ameliorating, alleviating, treating, or preventing diseases in humans or animals, that have milder effects than pharmaceuticals. For example, according to the Korean Pharmaceutical Affairs Act, a quasi-drug refers to articles other than those used as pharmaceuticals and includes products used in the treatment or prevention of diseases in humans or animals, products having mild effects on the human body, products that do not act directly on the human body, *etc*.

Another aspect of the present disclosure to achieve the above object provides a method for alleviating fatigue or enhancing exercise performance, comprising administering to a subject a composition comprising, as an active ingredient, a mixture of a mixed extract of angelica, cnidium, and paeonia and a polysaccharide isolate thereof; or a fraction thereof.

The mixed extract, polysaccharide isolate, mixture, and fraction are as described above.

As used herein, the term "administration" refers to introducing the composition of the present disclosure into a subject by any suitable method, and the administration route may be through various oral or parenteral routes, as long as the composition can reach a target tissue.

In the present disclosure, the subject may include a human or a non-human species, more specifically mammals including humans, and still more specifically humans, dogs, rats, rabbits, cats, horses, cows, *etc*. However, any subject may be included without limitation as long as fatigue alleviation or enhancement of exercise performance can be achieved by administration of the composition of the present disclosure.

Still another aspect of the present disclosure to achieve the above objects provides a use of a composition comprising, as an active ingredient, a mixture of a mixed extract of angelica, cnidium, and paeonia; and a polysaccharide isolate thereof for alleviating fatigue or enhancing exercise performance.

The mixed extract, polysaccharide isolate, mixture, and fraction are as described above.

Hereinafter, the present disclosure will be described in more detail with reference to Examples. However, these Examples are intended to exemplify the present disclosure, and the scope of the present disclosure is not limited by these Examples, as will be apparent to those skilled in the art to which the present disclosure pertains.

### Preparation Example. Preparation of composition

### Preparation Example 1. Preparation of mixture derived from angelica, cnidium, and paeonia

### Preparation Example 1-1. Preparation of mixed extract of angelica, cnidium, and paeonia

The root of angelica, rhizome of cnidium, and root of paeonia were each dried in the shade, cut into pieces, and mixed. Distilled water amounting to 10 times the total weight of each herbal medicinal ingredient (1,000 mL of distilled water per 100 g of herbal medicinal ingredient) was added, and hot water extraction was performed at 95°C for 4 hours to obtain a primary extraction liquid. In addition, using the residue of the first extraction, a second extraction liquid was obtained under the same conditions as the first extraction. The primary and secondary extracts were then combined, concentrated under reduced pressure, and filtered to obtain a mixed extract (see Table 1 below).

### Preparation Example 1-2. Preparation of polysaccharide isolate from mixed extract

A polysaccharide isolate was prepared from each mixed extract prepared in Preparation Example 1-1.

Specifically, to the mixed extract prepared in Preparation Example 1-1, 85% ethanol was added in an amount equal to four times the volume of the mixed extract. The mixture was left to stand at 25°C or less for 16 hours, and then centrifugation was performed to obtain a precipitated polysaccharide isolate from each mixed extract.

### Preparation Example 1-3. Preparation of mixture of mixed extract and polysaccharide isolate

A mixture in which the mixed extract prepared in Preparation Example 1-1 and the polysaccharide isolate prepared in Preparation Example 1-2 were mixed was prepared.

Specifically, each mixture was prepared by mixing each mixed extract prepared in Preparation Example 1-1 and each polysaccharide isolate prepared in Preparation Example 1-2. Thereafter, each of the prepared mixtures was filtered through sterile filter paper (Millipore membrane, 0.45 µm) to maintain a sterile state.

At this time, the mixed extract of Preparation Example 1-1 and the polysaccharide isolate of Preparation Example 1-2 were mixed at a weight ratio of 60:40.

The mixtures are all summarized as shown in Table 1 below.

**[Table 1]**

| Experimental group* | Angelica | Cnidium | Paeonia | Ratio |
|---|---|---|---|---|
| Example | *Angelica sinensis* (Oliv.) Diels | *Ligusticum chuanxiong* Hort. | *Paeonia lactiflora* Pallas | 1:1.6:1.3 |
| Comparative Example 1 | *Angelica gigas* Nakai | *Cnidium officinale* | *Paeonia lactiflora* Pallas | 1:1:1 |
| Comparative Example 2 | *Angelica sinensis* (Oliv.) Diels | *Ligusticum chuanxiong* Hort. | *Paeonia lactiflora* Pallas | 1:0.8:0.8 |
| Comparative Example 3 | *Angelica sinensis* (Oliv.) Diels | *Ligusticum chuanxiong* Hort. | *Paeonia lactiflora* Pallas | 1:4:1.3 |
| Comparative Example 4 | *Angelica sinensis* (Oliv.) Diels | *Ligusticum chuanxiong* Hort. | *Paeonia lactiflora* Pallas | 1:1.6:4 |
| Comparative Example 5 | *Angelica gigas* Nakai | *Cnidium officinale* | *Paeonia lactiflora* Pallas | 1:1.6:1.3 |
| Comparative Example 6 | *Angelica gigas* Nakai | *Ligusticum chuanxiong* Hort. | *Paeonia lactiflora* Pallas | 1:1.6:1.3 |

| | | | | |
|---|---|---|---|---|
| * Each experimental group is prepared in a form in which angelica, cnidium, and paeonia are mixed at the corresponding weight ratio and extracted, a polysaccharide isolate (ethanol polysaccharide isolate) of each extract is prepared, and then the prepared mixed extract and polysaccharide isolate are mixed. Table 1 does not describe the polysaccharide isolate but refers to a mixture form in which a polysaccharide isolate is mixed. | | | | |

### Experimental Example 1. Examination of expression level of antifatigue factors in vitro

### Experimental Example 1-1. Examination of expression level of antifatigue factors using L6 skeletal muscle cells

The effects of the composition of the present disclosure prepared in the above Preparation Example on muscle were evaluated.

Specifically, L6 skeletal muscle cells were seeded in a 6-well plate at a density of 4.5 x 10⁵ cells/well and incubated for 24 hours. The culture medium was replaced with a medium treated with the Example composition prepared in Preparation Example 1-3 at varying concentrations (31.3 µg/mL, 62.5 µg/mL, and 125 µg/mL), followed by incubation for 2 hours. Thereafter, citrinin, an oxidative stress-inducing substance, was treated at 60 µM, followed by further incubation for 24 hours. As a control group, a group treated with citrinin alone without treatment with the composition of the present disclosure was prepared. The cells were collected, and total RNA was extracted using an RNA extract kit (Cat. No. 74106, Qiagen, USA) according to the manufacturer's instructions. The extracted total RNA was used to prepare an RT-qPCR mixture using an RT-qPCR kit (TOPreal^{™} One-step RT qPCR kit, Cat. No. RT432M, Enzynomics, USA). An RT-qPCR mixture and the extracted RNA were dispensed into a 96-well plate, and RT-qPCR reactions and analyses were performed using a real-time PCR machine (CFX384, BIO-RAD, CA, USA) to determine the mRNA expression levels of Nrf-2, HO-1, SOD, CAT, Txn, Keap1, NQO1, GPx, Gclc, and GSr, respectively. All reactions were normalized to a housekeeping gene, and the results were averaged relative to a normal control group using the 2-ΔΔCT method.

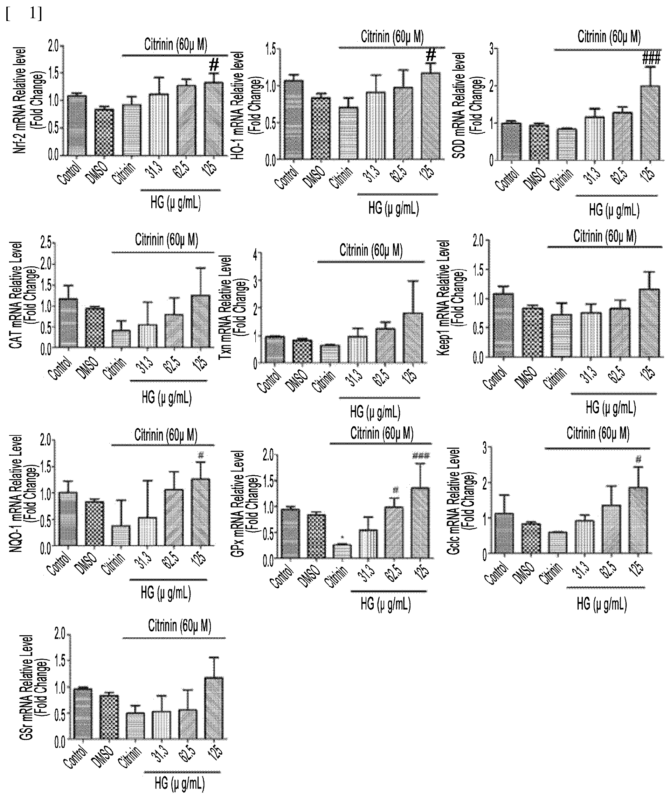

It was observed that as the treated concentration of the Example composition increased, the mRNA expression levels of antifatigue-related genes, including Nrf-2, HO-1, SOD, CAT, Txn, Keap1, NQO1, GPx, Gclc, and GSr, increased in the treated groups (see FIG. 1).

As such, it can be understood that use of the Example composition of the present disclosure results in an excellent antifatigue effect, thereby effectively alleviating fatigue or enhancing exercise performance.

### Experimental Example 1-2. Examination of antifatigue effect using C2C12 myotube cells

### Experimental Example 1-2-1. Examination of ATP and glycogen concentrations

The C2C12 mouse myoblasts used in this experiment were purchased from American Type Culture Collection (ATCC) and incubated using Dulbecco's Modified Eagle's Medium (DMEM) containing 10% fetal bovine serum (FBS), 100 U/mL penicillin, and 100 µg/mL streptomycin as a growth medium under 5% CO₂ and at 37°C. The C2C12 myoblasts were seeded in a 6-well plate at a density of 3 × 10⁵ cells/well and cultured. Thereafter, the cells were washed with PBS, the medium was replaced with a differentiation-inducing medium (DMEM containing 2% horse serum, 100 U/mL penicillin, and 100 µg/mL streptomycin), and the differentiation-inducing medium was replaced at 2-day intervals until differentiation. On day 6, differentiation was observed, and the Example composition was treated at varying concentrations (31.3 µg/mL, 62.5 µg/mL, and 125 µg/mL), while the compositions of Comparative Examples 1 to 6 were treated at 125 µg/mL. This was followed by incubation for 24 hours to obtain the cells. Thereafter, the contents of glycogen and ATP were measured using a glycogen assay kit (ab65620, abcam, USA) and an ATP assay kit (ab83355, abcam, USA), respectively.

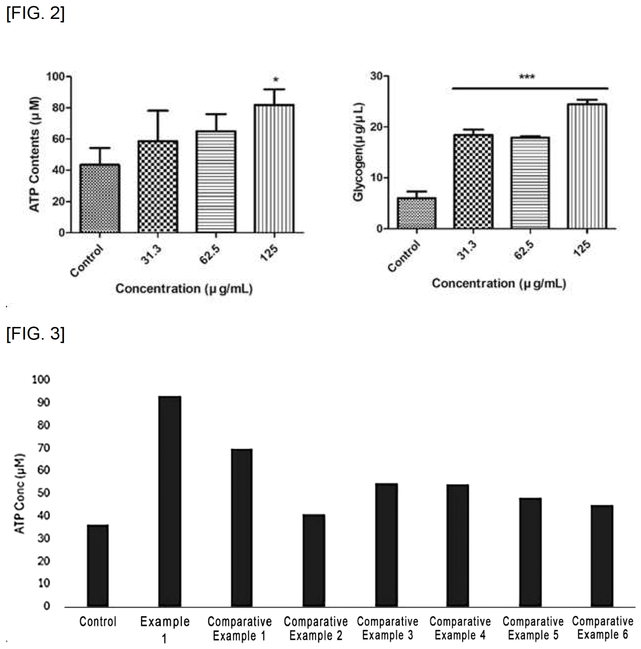

It was observed that as the treated concentration (31.3 µg/mL, 62.5 µg/mL, and 125 µg/mL) of the Example composition increased, the ATP and glycogen levels increased (see FIG. 2).

In addition, the group treated with the Example composition at a concentration of 125 µg/mL exhibited a significantly higher ATP content compared to the groups treated with the compositions of Comparative Examples 1 to 6 at the same concentration (see FIG. 3 and Table 2).

**[Table 2]**

| | Control | Example | Comparative Example 1 | Comparative Example 2 | Comparative Example 3 | Comparative Example 4 | Comparative Example 5 | Comparative Example 6 |
|---|---|---|---|---|---|---|---|---|
| ATP content (µM) | 55.82 | 93.32 | 70.05 | 51.34 | 54.52 | 54.34 | 51.34 | 50.46 |

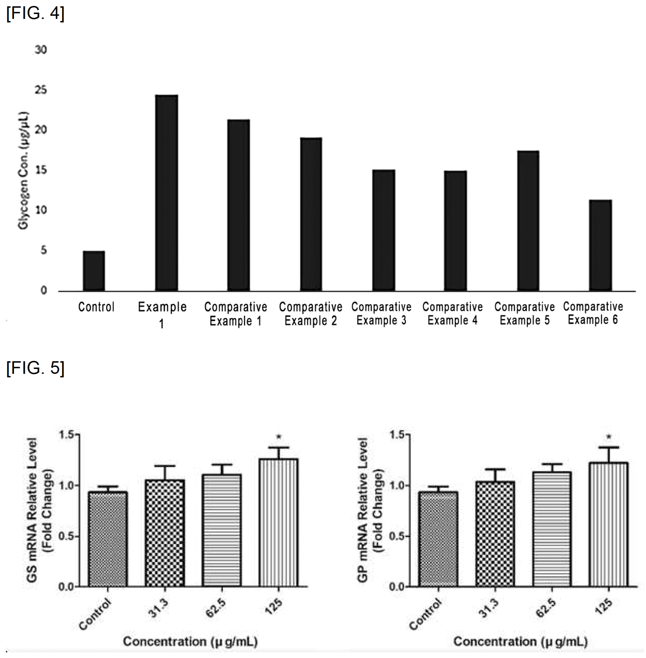

In addition, the group treated with the Example composition exhibited a significantly higher glycogen content compared to the groups treated with the compositions of Comparative Examples 1 to 6, thereby confirming a superior fatigue-alleviating effect (see FIG. 4 and Table 3).

**[Table 3]**

| | Control | Example | Comparative Example 1 | Comparative Example 2 | Comparative Example 3 | Comparative Example 4 | Comparative Example 5 | Comparative Example 6 |
|---|---|---|---|---|---|---|---|---|
| Glycogen content (µg/µL) | 5.02 | 26.32 | 21.38 | 20.3 | 18.2 | 17.5 | 19.5 | 15.45 |

As such, it can be understood that using the Example composition results in a superior fatigue-alleviating effect, or effectively increases exercise performance.

### Experimental Example 1-2-2. Examination of expression levels of glycogen synthase and glycogen phosphorylase

Next, from the cells obtained after 24 hours of incubation following treatment with the Example composition at varying concentrations (31.3 µg/mL, 62.5 µg/mL, and 125 µg/mL), total RNA was extracted using an RNA extract kit (Cat. No. 74106, Qiagen, USA) according to the manufacturer's instructions. The extracted total RNA was used to prepare an RT-qPCR mixture using an RT-qPCR kit, and analyses were performed to determine the mRNA expression levels of glycogen synthase (GS) and glycogen phosphorylase (GP), respectively. All reactions were normalized to a housekeeping gene, and the results were averaged relative to a normal control group using the 2-ΔΔCT method.

It was observed that the group treated with the Example composition exhibited increased expression levels of glycogen synthase and glycogen phosphorylase compared to the control group; as the treated concentration increased, the expression levels of glycogen synthase and glycogen phosphorylase increased (see FIG. 5).

As such, it can be understood that use of the Example composition of the present disclosure results in a superior fatigue-alleviating effect, or effectively increases exercise performance.

### Experimental Example 2. In vivo rotarod test and forced swimming test of a composition derived from natural products

### Experimental Example 2-1. Preparation of experimental mice

Using experimental mice, a rotarod test and a forced swimming test (FST) were performed.

6-week-old ICR mice (male) were used as the experimental mice. After acclimatization in a germ-free breeding device for one week, the mice were used in the experiment.

### Experimental Example 2-2. Rotarod test

The rotarod test was performed by conducting two training sessions during the first week of administration of the test substance (*i.e*., the Examples shown in Table 1), and then performing the test at a frequency of once per week after completion of the training. The test substance was administered at doses of 250 mg/kg and 500 mg/kg, respectively, 30 minutes prior to performing the rotarod test. As a positive control, creatine at a concentration of 300 mg/kg/day was administered, and as a negative control, mice (WT) to which no substance was administered were prepared. All mice were placed on lanes, and the rotation speed was accelerated from an initial speed of 4 rpm to 40 rpm over 10 minutes, and the time until exhaustion of each mouse was recorded.

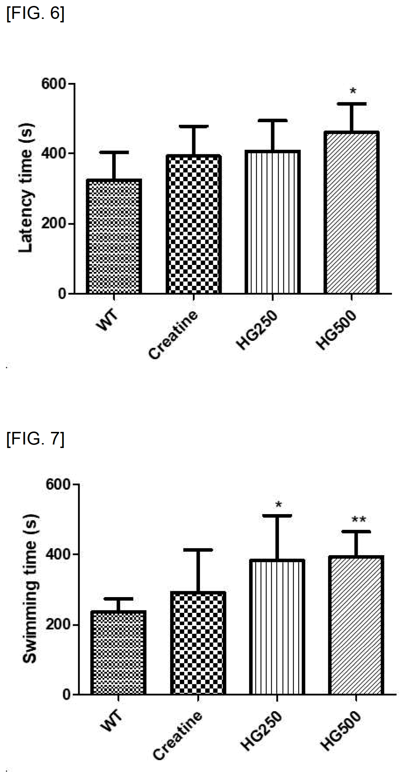

It was observed that the group administered with the Example composition not only exhibited increased exercise time compared to the control group, indicating enhanced exercise performance, but also showed effects comparable to or superior to those of the positive control group. In addition, it was observed that the exercise time increased as the treated dose of the Example composition increased (see FIG. 6).

As such, it can be understood that use of the Example composition of the present disclosure results in a superior fatigue-alleviating effect, and effectively increases exercise performance and endurance.

### Experimental Example 2-3. Forced swimming test (FST)

The forced swimming test was performed by conducting two training sessions during the first week of administration of the test substance (*i.e*., the Examples shown in Table 1), and then performing the test at a frequency of once per week after completion of the training. The test substance was administered at doses of 250 mg/kg and 500 mg/kg, respectively, 30 minutes prior to performing the FST. As a positive control, creatine at a concentration of 300 mg/kg/day was also administered. For forced swimming, a lead weight corresponding to approximately 10% of the body weight was attached to the tail of each mouse using paper tape, and the mouse was placed in water to undergo forced swimming. The time until exhaustion was measured.

It was observed that the group administered with the Example composition not only exhibited increased swimming time compared to the control group, indicating enhanced exercise performance, but also showed effects comparable to or superior to those of the positive control group. In addition, it was observed that the swimming time increased as the treated dose increased in the group administered with the Example composition (see FIG. 7).

As such, it can be understood that use of the Example composition of the present disclosure results in a superior fatigue-alleviating effect, and effectively increases exercise performance and endurance.

### Experimental Example 3. Measurement of changes in fatigue-related factors

### Experimental Example 3-1. Blood biochemical analysis

After completion of the forced swimming test in Experimental Example 2-3 above, test animals were anesthetized by inhalation of isoflurane, and blood samples were collected. The collected blood samples were placed in SST tubes and centrifuged at 3,000 rpm for 15 minutes to separate serum. Glucose and creatine kinase (CK) were analyzed using a blood biochemical analyzer.

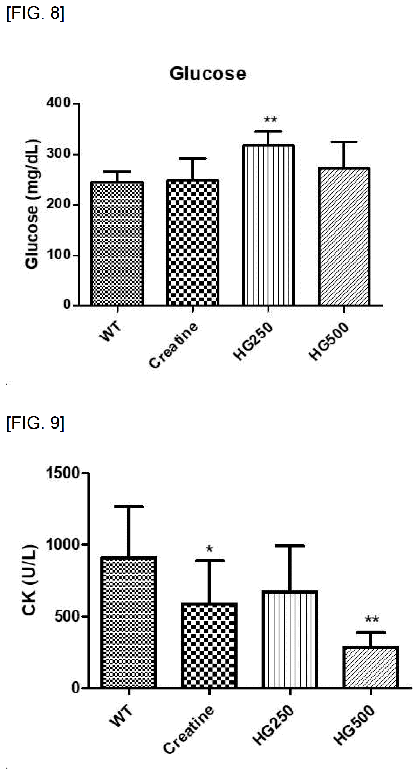

It was observed that blood glucose levels in the group administered with the Example composition significantly increased compared to those in the negative control group and positive control group (see FIG. 8), and that creatine kinase level significantly decreased (see FIG. 9).

### Experimental Example 3-2. Analysis of fatigue-related factors in tissues

After completion of the forced swimming test in Experimental Example 2-3 above, analysis of fatigue-related factors in muscle tissue of the test animals was performed using a glycogen assay kit (ab65620, abcam, USA) according to the manufacturer's instructions.

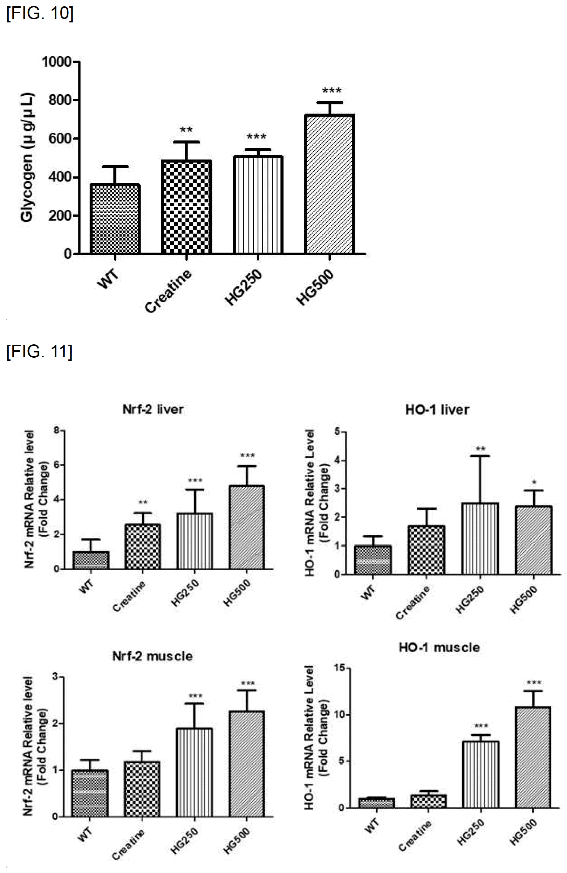

It was observed that glycogen content in muscle tissues increased as the treated dose increased in the group administered with the Example composition (see FIG. 10).

### Experimental Example 3-3. Analysis of antioxidant factors in tissues

After completion of the forced swimming test in Experimental Example 2-3 above, the mouse models were stored at -70°C, and RT-qPCR analysis was performed to analyze antioxidant factors in tissues.

Specifically, the stored mouse liver and soleus muscle tissues were each aliquoted to 30 mg and homogenized with RLT buffer (Cat. No. 79216, Qiagen, USA) and beads using a Beadruptor (BEAD RUPTOR 24, OMNI Inc., USA). Centrifugation (4°C, 13,000 rpm, 3 minutes) was performed and the supernatant was removed, then total RNA was extracted using an RNA extract kit (Cat. No. 74106, Qiagen, USA) according to the manufacturer's instructions. A kit (TOPreal^{™} One-step RT qPCR kit, Cat. No. RT432M, Enzynomics, USA) was used to prepare an RT-qPCR mixture, then RT-qPCR reactions and analyses were performed using a real-time PCR machine (CFX384, BIO-RAD, CA, USA). All reactions were normalized to a housekeeping gene, and the results were averaged relative to a normal control group using the 2-ΔΔCT method.

It was observed that, in both liver and muscle, administration of the Example composition increased the expression levels of Nrf-2 and HO-1 compared to the control group, and the expression levels gradually increased as the administered dose of the Example composition increased (see FIG. 11).

In addition, western blot analysis was performed for analysis of antioxidant factors in tissues.

Specifically, liver and calf muscle (gastrocnemius) tissues excised from the stored mice were homogenized by adding RIPA buffer (Cat. No. 89901, Thermo Scientific, U.S.A), followed by centrifugation (14,000 rpm, 4°C, 10 minutes), and the supernatant was obtained. Thereafter, protein was quantified using the bicinchoninic acid (BCA) method, and 50 µg of the protein was subjected to electrophoresis on 10% Tris-Glycine Mini Protein Gels (Cat. No. XP00102BOX, Invitrogen, USA). After completion of electrophoresis, the gel was transferred onto a PVDF membrane using a Power Blotter System (Cat. No. PB0010, Invitrogen, U.S.A). The membrane was blocked with 5% skim milk for 1 hour and then washed. A secondary antibody (7074S, Cell Signaling, USA) was diluted 1:1,000 in 5% skim milk and reacted for 1 hour, after which the PVDF membrane was treated with an ECL substrate and imaged using ImageQuant.

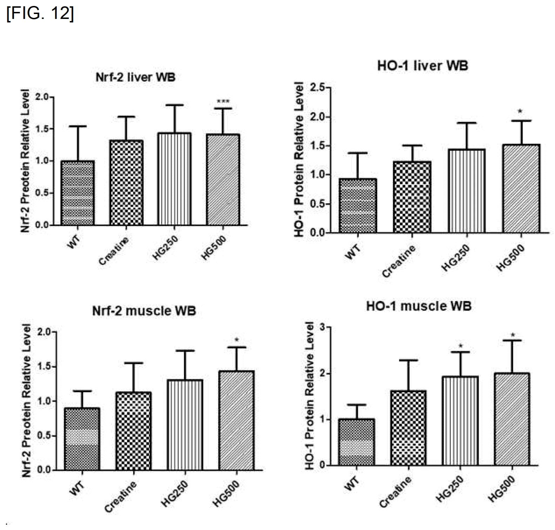

It was observed that, in both liver and muscle, administration of the Example composition increased the protein expression levels of Nrf-2 and HO-1 compared to the control group (see FIG. 12).

### Experimental Example 3-4. Immunohistochemical staining analysis of tissue

After completion of the forced swimming test in Experimental Example 2-3 above, excised calf muscle (gastrocnemius) tissues from the mouse models stored at -70°C were prepared as slides, dried at room temperature, and fixed by acetone treatment. After blocking with 5% normal goat serum, a primary antibody against Nrf-2 (Cat. No. NBP1-32822, Novus, USA) was diluted 1:1,000 using 5% normal goat serum and incubated overnight at 4°C. A secondary antibody (BA1003, BOSTER, USA) was diluted 1:1,000 in 5% normal goat serum, treated for 1 hour, and then washed with 1 x PBS. Thereafter, color development was performed by reacting with a DAB staining solution (SK-4105, Vector Laboratories, USA) for 30 seconds, followed by mounting with a mounting medium containing DAPI (Cat. No. H-1200-10, Vector, United States), and observation was conducted using a fluorescence microscope. The area exhibiting brown coloration due to staining within the same region was calculated using the ImageJ program.

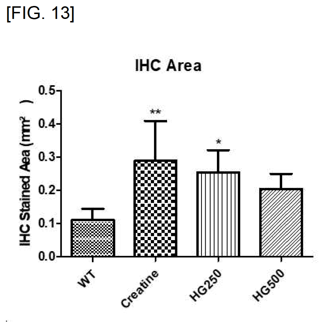

It was observed that the group administered with the Example composition exhibited a broader stained area than the negative control group in which no treatment was applied (see FIG. 13). These results visually confirm the expression level of the antioxidant factor Nrf-2 in tissues through immunohistochemical staining (IHC) and quantitatively analyze the same. A broader stained area indicates increased expression of Nrf-2, which demonstrates that the group administered with the Example composition exhibits a superior antioxidant effect compared to the control group.

Based on the above description, it will be understood by those skilled in the art that the present disclosure may be implemented in a different specific form without changing the technical spirit or essential characteristics thereof. Therefore, it should be understood that the above embodiment is not limitative, but illustrative in all aspects. The scope of the disclosure is defined by the appended claims rather than by the above description, and therefore all changes and modifications that fall within the scope of the claims or equivalents of such scope are therefore intended to be embraced by the claims.

## Claims

1. A composition for alleviating fatigue or enhancing exercise performance, comprising, as an active ingredient, a mixture of a mixed extract of angelica, cnidium, and paeonia; and a polysaccharide isolate thereof.

2. The composition according to claim 1, wherein the fatigue comprises physical fatigue, muscle fatigue, or a combination thereof.

3. The composition according to claim 1, wherein the mixed extract is extracted with a solvent selected from the group consisting of water, an organic solvent, and a mixed solvent thereof.

4. The composition according to claim 1, wherein the mixed extract comprises angelica, cnidium, and paeonia at a weight ratio of 1:1.4 to 3.5:1.2 to 3.

5. The composition according to claim 1, wherein the mixed extract is a mixed extract of *Angelica sinensis* (Oliv.) Diels, *Ligusticum chuanxiong* Hort. or *Cnidium officinale*, and *Paeonia lactiflora* Pallas.

6. The composition according to claim 1, wherein the polysaccharide isolate is an ethanol polysaccharide isolate.

7. The composition according to claim 6, wherein the ethanol is 60% to 100% ethanol.

8. The composition according to claim 1, wherein the mixture comprises the mixed extract of angelica, cnidium, and paeonia and the polysaccharide isolate thereof at a ratio of 60 to 70:30 to 40.

9. The composition according to claim 1, wherein the mixture is comprised in an amount of 0.001% to 90% by weight based on the total weight of the composition.

10. The composition according to claim 1, wherein the composition increases expression of heme oxygenase-1 (HO-1).

11. The composition according to claim 1, wherein the composition activates a nuclear factor erythroid 2-related factor-2 (Nrf-2) pathway.

12. The composition according to claim 1, wherein the composition increases expression of one or more selected from the group consisting of SOD, CAT, Txn, Keap1, NQO1, GPx, Gclc, and GSr.

13. The composition according to claim 1, wherein the composition increases content of one or more selected from the group consisting of ATP and glycogen.

14. The composition according to claim 1, wherein the composition increases expression of one or more selected from the group consisting of glycogen synthase and glycogen phosphorylase.

15. The composition according to claim 1, wherein the composition is a food composition.

16. The composition according to claim 15, wherein the food composition is prepared as a powder, granule, tablet, capsule, syrup, or beverage.

17. The composition according to claim 15, wherein the food is a health functional food.

18. A method for alleviating fatigue or enhancing exercise performance, comprising administering to a subject a composition comprising, as an active ingredient, a mixture of a mixed extract of angelica, cnidium, and paeonia; and a polysaccharide isolate thereof.

19. Use of a composition comprising, as an active ingredient, a mixture of a mixed extract of angelica, cnidium, and paeonia; and a polysaccharide isolate thereof, for alleviating fatigue or enhancing exercise performance.
